# EUROPÄISCHE PATENTANMELDUNG

(11) **EP 0 592 814 A2**
(43) Veröffentlichungstag der Anmeldung: **20.04.1994**
(21) Anmeldenummer: 93114536.1
(22) Anmeldetag: 10.09.1993
(51) Int. Cl.: A61M 5/32

(54) **Verfahren zur Herstellung einer Nadelschutzkappe sowie Nadelschutzkappe zum Aufstecken oder Aufschieben auf eine Spritznadel**

(30) Priorität: 12.10.1992 DE 4234319
(71) Anmelder: Pharma-Gummi Wimmer West GmbH, D-52249 Eschweiler (DE)
(72) Erfinder: Becker, Bernd, Dipl.-Ing., B-4721 Kelmis (DE)
(74) Vertreter: Patent- und Rechtsanwaltssozietät, Schmitt, Maucher & Börjes

(57) **Zusammenfassung**

Die Erfindung betrifft ein Verfahren zur Herstellung einer Nadelschutzkappe sowie eine Nadelschutzkappe (1) zum Aufstecken oder Aufschieben auf eine Spritze, mit mindestens zwei Teilbereichen, von denen ein erster Teilbereich als steifer hülsenförmiger Stützkörper (4) ausgebildet ist und von denen wenigstens ein zweiter, aus elastischem Material bestehender Teilbereich (5, 6) im Hülseninneren des Stützkörpers (4) angeordnet ist und in Gebrauchsstellung zumindest bereichsweise die Nadel und/oder den Spritzenkörper dicht umgreift, wobei diese beiden Teilbereiche (4; 5, 6) der Nadelschutzkappe (1) fest miteinander verbunden sind. Dabei ist kennzeichnend für die erfindungsgemäße Nadelschutzkappe (1), daß der als Stützkörper (4) vorgesehenen Teilbereich aus einem thermoplastischen Kunststoff und der elastische Teilbereich (die elastischen Teilbereiche) (5, 6) aus einem thermoplastischen Elastomer bestehen und daß diese Teilbereiche (4; 5, 6) der Nadelschutzkappe (1) untrennbar miteinander verschweißt sind.

## Beschreibung

Die Erfindung betrifft ein Verfahren zur Herstellung einer Nadelschutzkappe, die zumindest zwei Teilbereiche aus gegebenenfalls unterschiedlichen Materialien oder Formmassen aufweist, von denen ein Teilbereich der Nadelschutzkappe einen im wesentlichen steifen Stützkörper bildet und von denen wenigstens ein anderer Teilbereich aus elastischem Material die Nadel und/oder den Spritzenkörper dicht umgreift.

Die Erfindung befaßt sich auch mit einer Nadelschutzkappe zum Aufstecken oder Aufschieben auf die Nadel und/oder den Spritzenkörper einer Spritze, mit mindestens zwei Teilbereichen, von denen ein erster Teilbereich als steifer hülsenförmiger Stützkörper ausgebildet ist und von denen wenigstens ein zweiter, aus elastischem Material bestehender Teilbereich im Hülseninneren des Stützkörpers angeordnet ist und in Gebrauchsstellung zumindest bereichsweise die Nadel und/oder den Spritzenköper dicht umgreift, wobei diese beiden Teilbereiche der Nadelschutzkappe fest miteinander verbunden sind.

Aus der FR-2 644 069 A1 kennt man bereits eine Nadelschutzkappe, die zwei Teilbereiche aufweist, von denen ein erster Teilbereich als steifer hülsenförmiger Stützkörper ausgebildet ist und von denen ein zweiter Teilbereich aus elastischem Material hergestellt ist. Dieser zweite elastische Teilbereich ist im Hülseninneren des einseitig offenen Stützkörpers vorgesehen und nimmt in Gebrauchsstellung die Nadel einer Spritze im Bereich ihrer Nadelspitze auf. Während also der elastische Teilbereich die Nadel zumindest bereichsweise umgreift und die Spritzenöffnung an der Nadelspitze dicht verschließt, bietet der harte Stützkörper Schutz gegen unbeabsichtigte Verletzungen an der Spritzennadel, die ansonsten eventuell auch zu gefährlichen Infektionen führen könnten.

Da die beiden, aus unterschiedlichem Material bestehenden Teilbereiche dieser vorbekannten Nadelschutzkappe getrennt voneinander hergestellt und anschließend fest miteinander verklebt werden, ist zu dessen Herstellung noch ein vergleichsweise hoher Aufwand erforderlich.

Es besteht daher insbesondere die Aufgabe, ein Verfahren der eingangs erwähnten Art zu schaffen, daß die Herstellung einer zumindest aus zwei Teilbereichen bestehenden Nadelschutzkappe mit vergleichsweise geringem Aufwand erlaubt. Darüber hinaus besteht auch die Aufgabe, eine Nadelschutzkappe der oben erwähnten Art zu schaffen, die mit vergleichsweise wenig Aufwand herstellbar ist.

Die erfindungsgemäße Lösung dieser Aufgabe besteht bei dem Verfahren der eingangs erwähnten Art insbesondere darin, daß beide Teilbereiche der Nadelschutzkappe im Spritzgußverfahren in zumindest zwei Verfahrensschritten hergestellt werden, daß dazu in einem ersten Verfahrenschritt ein erster Teilbereich der Nadelschutzkappe hergestellt wird und daß in einem der Nadelschutzkappe hergestellt wird und daß in einem nachfolgenden Verfahrenschritt zumindest ein zweiter Teilbereich hergestellt wird, wobei beim Einspritzen der Formmasse für den zweiten Teilbereich in oder auf den ersten Teilbereich der Nadelschutzkappe dieser mit dem zweiten Teilbereich fest und untrennbar verschweißt oder dergleichen verbunden wird.

Bei dem erfindungsgemäßen Verfahren wird die Nadelschutzkappe im Spritzgußverfahren in zumindest zwei Verfahrensschritten hergestellt. Dabei werden die für die beiden Teilbereiche jeder Nadelschutzkappe vorgesehenen Formmassen erhitzt, plastifiziert und mit einer entsprechenden Temperatur in die formgebende Höhlung des Spritzgießwerkzeuges gespritzt. Da auch die Formmasse für den zweiten Teilbereich der Nadelschutzkappe im Spritzwerkzeug mit einer hohen Temperatur in oder über den ersten Teilbereich gespritzt wird, wird das mit dieser heißen Formmasse in Kontakt kommende thermoplastische Material des ersten und bereits in einem vorangegangenen Verfahrensschritt in seine Form gebrachten Teilbereiches zumindest oberflächlich derart erhitzt und plastifiziert, daß die beiden aus dem selben oder vorzugsweise aus unterschiedlichen thermoplastischen Materialien bestehenden Teilbereiche der erfindungsgemäßen Nadelschutzkappe fest und untrennbar miteinander verschweißen. Mit Hilfe des erfindungsgemäßen Verfahrens können somit Nadelschutzkappen der eingangs erwähnten Art auch in großem Umfang mit vergleichsweise geringem Aufwand hergestellt werden.

Dabei wird ein Verfahren bevorzugt, bei dem in einem ersten Verfahrensschritt der Stützkörper hergestellt wird.

Um auch die Vorteile unterschiedlicher Werkstoffe für die verschiedenen Teilbereiche einer Nadelschutzkappe nutzen zu können, ist es Zweckmäßig, wenn die Nadelschutzkappe im Zwei-Komponenten-Spritzgußverfahren hergestellt wird.

Die erfindungsgemäße Lösung bei der Nadelschutzkappe der eingangs erwähnten Art besteht insbesondere darin, daß der als Stützkörper vorgesehene Teilbereich aus einem thermoplastischen Kunststoff und der elastische Teilbereich (die elastischen Teilbereiche) aus einem thermoplastischen Elastomer betehen und daß diese Teilbereiche der Nadelschutzkappe untrennbar miteinander verschweißt sind.

Die Teilbereiche der erfindungsgemäßen Nadelschutzkappe bestehen aus unterschiedlichen thermoplastischen Materialien und sind fest miteinander verschweißt. Durch diese untrennbare Verbindung eines die Nadelschutzkappe aussteifenden Stützkörpers mit zumindest einem Teilbereich aus elastischem Material, welcher praktisch als Dichtelement die Nadel und/oder den Spritzenkörper eiener Spritze dicht umgreift, läßt sich ein steifes Bauteil schaffen, das mit geringem Aufwand herstellbar ist sowie exakt zu montieren ist und auch bei hohen Taktzahlen während der Herstellung der Spritzen nur vergleichsweise wenig zu Störungen in den Herstellungs- und Füllmaschinen neigt. Dadurch wir eine schnelle, sichere und kostengünstige Verarbeitung dieser Kappe auch beim Spritzenhersteller begünstigt. Zudem läßt sich mit der untrennbaren Verbindung der verschiedenen Teilbereiche auch eine Nadelschutzkappe schaffen, die hohen hygienischen Anforderungen genügt und mit geringem Aufwand zu sterilisieren ist. Auch ohne Trennung der thermoplastischen Materialien ist die Werkstoffkombination der erfindungsgemäßen Nadelschutzkappe gut recyclebar.

Der im Stützkörper als Dichtelement vorgesehene Teilbereich vermag auch solche Spritzen gut abzudichten, die bereits vollständig fertig konfektioniert, d.h. mit einer am Spritzenkörper montierten Kanüle und gegenbenenfalls auch bereits mit einem Medikament befüllt, in den Handel kommen. Dabei ist es besonders vorteilhaft, wenn der Stützkörper in seinem Hülseninneren einen elastischen Teilbereich trägt, der die Nadel zumindest im Bereich der Nadelspitze und/oder den Spritzenkörper in seinem an die Nadel angrenzenden Bereich dicht umgreift.

Die Nadelschutzkappe läßt sich besonders einfach und dennoch dicht auf eine solche Spritze aufsetzen, wenn die Nadelschutzkappe einen Kappeninnenraum mit einer Einstecköffnung hat, welche an einer Stirnseite der Nadelschutzkappe angeordnet ist und wenn der Kappeninnenraum zumindest bereichsweise an die Nadel und/oder den Spritzenkörper formangepaßt ist.

Der Stützkörper der erfindungsgemäßen Nadelschutzkappe steift nicht nur deren als Dichtelement vorgesehenen Teilbereich aus, vielmehr verhindert der Stützkörper auch, daß die Nadel beim Aufsetzen der Nadelschutzkappe diese unbeabsichtigt durchsticht und die Person verletzt oder infiziert, die beispielsweise nach einer Applikation am Patienten die Nadelschutzkappe auf die Spritzennadel aufschieben will. Dadurch wird ein Infektionsrisiko beispielsweise mit Hepatitis- oder AIDS-Erregern erheblich reduziert. Um ein solches Durchstechen der Nadelschutzkappe praktisch über die gesamte Längserstreckung der Kappenseitenwand auszuschließen, ist es zweckmäßig, wenn der Stützkörper in Gebrauchsstellung der Nadelschutzkappe mit der Nadelspitze abschließt oder diese überragt.

Auch eine fertig konfektionierte und mit einem Medikament befüllte Spritze läßt sich besonders gut und dicht verschließen, wenn der hülsenförmige Stützkörper an seiner der Einstecköffnung abgewandten Stirnseite von einem elastischen Teilbereich dicht verschlossen ist, welcher vorzugsweise über das der Einstecköffnung abgewandte Stirnende des Stützkörpers zumindest bereichsweise übersteht.

Das Aufsetzen der Nadelschutzkappe auf die Nadel sowie das Nadelansatzstück des Spritzenkörpers wird erleichtert, wenn die Einstecköffnung durch einen elastischen Teilbereich begrenzt ist, der vorzugweise über das der Einstecköffnung zugewandte Stirnende des Stützelementes übersteht.

Dabei wird das leichte Aufsetzen der erfindungsgemäßen Nadelschutzkappe auf eine Spritzennadel noch begünstigt, wenn der Kappeninnenraum sich vorzugsweise in seinem zwischen einem Nadelansatzstück des Spritzenkörpers und der Nadelspitze angeordneten Abschnitt in Einsteckrichtung zumindest bereichsweise konisch verjüngt und wenn der Kappeninnenraum einen weiteren Abschnitt mit im wesentlichen gleichbleibendem Querschnitt hat, welcher die Nadel im Bereich der Nadelspitze dicht umgreift. Beim Auschieben der Nadelschutzkappe auf die Nadel wird letztere nämlich zunächst in dem sich in Einsteckrichtung konisch verjüngenden Abschnitt des Kappeninnenraums praktisch geführt und zentriert, um anschließend in Gebrauchsstellung der Nadelschutzkappe von deren Abschnitt mit gleichbleibendem Querschnitt dicht umgriffen zu werden.

Um die Nadelschutzkappe fest und dicht in ihrer Gebrauchsstellung auf der Spritzennadel und/oder dem Spritzenkörper zu halten, ist es zweckmäßig, wenn im Kappeninnenraum der Nadelschutzkappe ein vorzugsweise umlaufender innerer Rastvorsprung vorgesehen ist, der in Gebrauchsstellung in eine Einformung der Spritze eingreift und/oder diese Einformung hintergreift. Dabei sieht eine besonders einfache und mit geringem Aufwand herstellbare Ausführungsform gemäß der Erfindung vor, daß der Rastvorsprung als Ausformung eines elastischen Teilbereichs der Nadelschutzkappe und die damit zusammenwirkende Einformung der Spritze als eine vorzugsweise an die Nadel angrenzende und insbesondere wulstförmige Querschnittserweiterung des Nadelansatzstückes ausgebildet ist.

Da ein Teilbereich der Nadelschutzkappe, der aus elastischem Material besteht, von der Nadelspitze leicht angestochen oder durchstochen werden kann, ist es vorteilhaft, wenn der konisch verjüngte Abschnitt des Kappeninnenraums durch eine vorzugsweise flanschartige Einformung im Hülseninneren des Stützkörpers gebildet ist und wenn diese Einformung des Stützkörpers in seinem Hülseninneren zwei elastische Teilbereiche voneinander trennt. Denn insbesondere in einem aus hartem oder steifem Material bestehenden und sich in Einsteckrichtung konisch verjüngenden Abschnitt wird die Spritzennadel beim Aufschieben der Nadelschutzkappe gut geführt.

Dabei ist es zweckmäßig, wenn der Stützkörper in seinem Hülseninneren mit zwei elastischen Teilbereichen untrennbar verschweißt ist, von denen ein erster Teilbereich den Spritzenkörper vorzugsweise im Bereich des Nadelansatzstückes und von denen ein zweiter Teilbereich die Nadel zumindest bereichsweise dicht umgreift und wenn diese beiden elastischen Teilbereiche vorzugsweise über einen aus demselben thermoplastischen Elastomer bestehenden Außenmantel miteinander verbunden sind, welcher den Stützkörper außenseitig umgibt. Bei einer solchen Ausführungsform gemäß der Erfindung ist der Stützkörper in die elastischen Teilbereiche der erfindungsgemäßen Nadelschutzkappe praktisch integriert.

Demgegenüber sieht eine bevorzugte Ausführungsform gemaß der Erfindung vor, daß die Nadelschutzkappe einen einstückigen elastischen Teilbereich aufweist und daß das Stützelement diesen Teilbereich zumindest bereichsweise hülsenförmig umgreift und vorzugsweise im wesentlichen den Außenmatel der Nadelschutzkappe bildet. Insbesondere eine solche Ausführungsform gemäß der Erfindung begünstigt den geringen Aufwand bei der Herstellung der erfindungsgemäßen Nadelschutzkappe.

Weitere Merkmale der Erfindung ergeben sich aus der vorliegenden Beschreibung erfindungsgemäßer Ausführungsbeispiele in Verbindung mit den Ansprüchen sowie der Zeichnung. Die einzelnen Merkmale können je für sich oder zu mehreren bei einer Ausführungsform der Erfindung verwirklicht sein.

Es zeigt in unterschiedlichen Maßstäben:
- **Fig.1**: eine Nadelschutzkappe in einem Längsschnitt, die mehrere Teilbereiche aufweist, von denen ein Teilbereich als steifer hülsenförmiger Stützkörper vorgesehen ist, welcher im Bereich seiner beiden Stirnenden innenseitig jeweils mit einem Teilbereich aus elastischem Material fest verschweißt ist,
- **Fig.2**: die Nadelschutzkappe aus Figur 1 in ihrer ebenfalls längsgeschnittenen Gebrauchsstellung, in der sie auf eine Spritze aufgeschoben ist und deren Spritzennadel sowie den Spritzenkörper bereichsweise dicht umgreift,
- **Fig. 3**: eine Nadelschutzkappe, ähnlich der aus Figur 1, ebenfalls in einem Längsschnitt,
- **Fig. 4**: eine Nadelschutzkappe in einem Längsschnitt, die aus zwei Teilbereichen besteht, von denen ein erster Teilbereich aus elastischem Material einen Kappeninnenraum zur Aufnahme der Spritze begrenzt und von denen ein zweiter, als steifer Stützkörper ausgebildeter Teilbereich zumindest bereichsweise den ringförmigen Außenmantel der Nadelschutzkappe bildet und
- **Fig. 5**: die Nadelschutzkappe aus Figur 4 in ihrer Gebrauchsstellung.

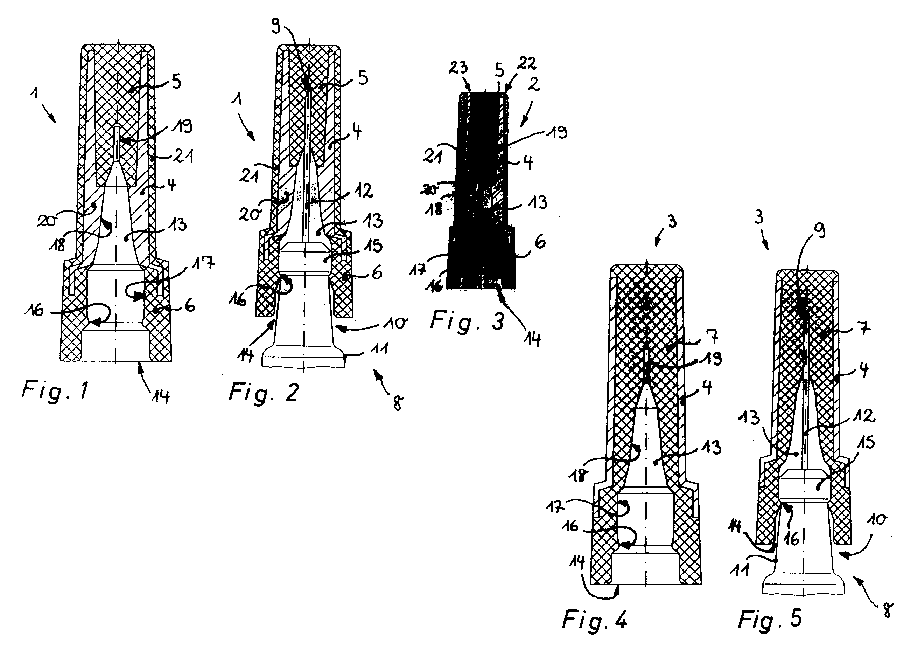

Die in den Figuren 1 bis 5 dargestellten Nadelschutzkappen 1, 2 und 3 weisen zumindest zwei Teilbereiche auf, von denen ein erster Teilbereich als steifer hülsenförmiger Stützkörper 4 ausgebildet ist, der in seinem Hülseninneren mit wenigstens einem zweiten, aus elastischem Material bestehenden Teilbereich 5, 6 bzw. 7 fest und untrennbar verschweißt ist. In der beispielsweise in den Figuren 2 und 5 dargestellten Gebrauchsstellung der Nadelschutzkappen 1 ,2 und 3 umgreifen deren elastischen Teilbereiche 5, 6 bzw 7 die Spritzen 8 bereichsweise und liegen im Bereich der Nadelspitze 9 sowie dem daran angrenzenden Nadelansatzstück 10 der Spritzenkörper 11 an.

Zur Aufnahme der Spritzen 8 im Bereich der Spritzennadel 12 weisen die Nadelschutzkappen 1, 2 und 3 einen Kappeninnenraum 13 mit einer Einstecköffnung 14 auf, der bereichsweise an die Nadelspitze 9 sowie an eine wulstförmige Querschnittserweiterung 15 der Nadelansatzstücke 10 formangepaßt ist. Die elastischen Teilbereiche 5 bzw. 7 der Nadelschutzkappen 1, 2 und 3 schließen das der Einstecköffnung 14 abgewandte stirnseitige Ende der Stützkörper 4 dicht ab, so daß auch die Nadelspitze fertig vorkonfektionierter und beispielsweise mit einem Medikament vorgefüllter Spritzen 8 oder medizinischer Einmalspritzen dicht abgeschlossen ist. Wie die Figuren 1 bis 5 zeigen, stehen die elastischen Teilbereiche 5, 7 der Spritzen 8 zumindest bereichsweise geringfügig über das der Einstecköffnung abgewandte Stirnende des Stützkörpers 4 über.

Die hülsenförmigen Stützkörper 4 der in den Figuren 1 bis 5 dargestellten Nadelschutzkappen 1, 2, 3 überragen in Gebrauchsstellung dieser Nadelschutzkappen die Nadelspitzen der in ihren Hülseninneren angeordneten Nadeln 12, so daß ein unbeabsichtigtes Durchstechen der elastischen Teilbereiche 5, 6, 7 an der Kappenseitenwand und eine Verletzung des Bedienungspersonals weitestgehend vermieden wird.

Die Nadelschutzkappen 1, 2, 3 weisen in ihrem Kappeninnenraum 13 mit Abstand von der Einstecköffnung 14 einen umlaufenden inneren Rastvorsprung 16 auf, der in Gebrauchsstellung der Nadelschutzkappen 1, 2, 3 eine Einformung der Spritze 9 hintergreift, die durch die an die Nadel angrenzende, wulstförmige Querschnittserweiterung 15 des Nadelansatzstückes 10 gebildet ist. Dabei ist der Rastvorsprung 16 als Ausformung des der Einstecköffnung 14 zugewandten elastischen Teilbereichs 6 bzw. 7 ausgebildet.

Während der Kappeninnenraum 13 in seinem in Einsteckrichtung unmittelbar hinter dem Rastvorsprung 16 angeordneten und als innere Ringnut 17 ausgebildeten Abschnitt an den Außendurchmesser der Querschnittserweiterung 15 des Nadelansatzstückes 10 formangepaßt ist und daran dicht anliegt, hat er in seinem die Einstecköffnung begrenzenden Bereich einen im Vergleich zum Außendurchmesser des in Gebrauchsstellung benachbarten Bereichs des Nadelansatzstückes 10 geringfügig größeren lichten Durchmesser, was das Aufsetzen der Nadelschutzkappen 1, 2 und 3 auf die Spritzennadel 12 erleichtert.

Der Abschnitt 18 des Kappeninnenraums 13, der in Gebrauchsstellung zwischen dem Nadelansatzstück 10 des Spritzenkörpers 11 und der Nadelspitze 9 im Anschluß an die Ringnut 17 angeordnet ist, verjüngt sich in Einsteckrichtung bereichsweise konisch und geht unmittelbar in einen endseitig ge weiteren Abschnitt 19 des Kappeninnenraums 13 mit im wesentlichen gleichbleibendem Querschnitt über, welcher die Nadel 12 im Bereich der Nadelspitze 9 dicht umgreift. Beim Auschieben der Nadelschutzkappen 1, 2 und 3 auf eine Spritzennadel 12 werden die Nadeln in diesem Bereich geführt und zu dem durch den elastischen Teilbereich gebildeten Abschnitt 19 hin zentriert, der die jeweilige Spritze 8 in Gebrauchsstellung dicht abschließt.

Dabei wird der sich konisch verjüngende Abschnitt 18 des Kappeninnenraums 13 bei den in Figur 1 bis 3 dargestellten Spritzen 1 und 2 durch eine flanschartige Einformung 20 des Stützkörpers 4 gebildet, die in seinem Hülseninneren die elastischen Teilbereiche 5 und 6 voneinander trennt, wobei der Teilbereich 6 den Spritzenkörper 11 im Bereich der Querschnittserweiterung 15 des Nadelansatzstückes 10 und der elastische Teilbereich 5 die Spritzennadel 12 im Bereich ihrer Nadelspitze 9 dicht umgreift. Dabei sind die elastischen Teilbereiche 5 und 6 über einen Außenmantel 21 miteinander verbunden, welcher den Stützkörper 4 außenseitig umgibt und aus dem selben thermoplastischen Elastomer wie die Teilbereiche 5 und 6 besteht.

Demgegenüber weist die in den Figuren 4 und 5 dargestellte Nadelschutzkappe 3 nur einen einstückigen und in Gebrauchsstellung an der Nadelspitze 9 sowie der Querschnittserweiterung 15 anliegenden Teilbereich 7 auf, den das Stützelement über einen Abschnitt seiner Längserstreckung bereichsweise hülsenförmig umgreift. Bei dieser Ausführungsform ist das Stützelement 4 nicht in das elastische Material der Nadelschutzkappe integriert, sondern bildet im wesentlichen deren Außenmantel.

Die hier dargestellten Nadelschutzkappen können mit geringem Aufwand in einem Zwei-Komponenten-Spritzgußverfahren hergestellt werden. Dazu wird in einem ersten Verfahrensschritt zunächst der aus einem thermoplastischen Kunststoff bestehende Stützkörper 4 hergestellt. In einem nachfolgenden Verfahrensschritt werden sodann die aus einem thermoplastischen Elastomer bestehenden Teilbereiche 5, 6 bzw. 7 der Nadelschutzkappen 1, 2, 3 hergestellt, wobei beim Einspritzen der heißen Formmasse für die elastischen Teilbereiche auch der thermoplastische Kunststoff des Stützkörpers 4 zumindest oberflächlich erhitzt und vorübergehend aufgeweicht wird. Nach dem Erkalten und Aushärten der Nadelschutzkappe 1, 2, 3 sind die thermoplastischen Materialien ihrer verschiedenen Teilbereiche fest und untrennbar verschweißt.

Der Stützkörper 4 der in Figur 3 dargestellten Nadelschutzkappe 2 weist an seinem der Einstecköffnung 14 abgewandten Stirnende zumindest eine, vorzugsweise mehrere randseitig offene Aussparungen 22 auf, die eine gleichmäßige Verteilung des für den Außenmatel 21 sowie die Teilbereiche 5 und 6 vorgesehenen thermoplastischen Elastomers während des Spritzgußverfahrens erleichtert.Dabei bilden die zwischen den Aussparungen 22 verbleibenden Vorsprünge 23 während des Spritzvorgangs steife Auflagepunkte im Spritzgießwerkzeug.

## Patentansprüche

1. Verfahren zur Herstellung einer Nadelschutzkappe, die zumindest Zwei Teilbereiche aus gegebenenfalls unterschiedlichen Materialien oder Formmassen aufweist, von denen ein Teilbereich der Nadelschutzkappe einen im wesentlichen steifen Stützkörper bildet und von denen wenigsten ein anderer Teilbereich aus elastischem Material die Nadel und/oder den Spritzenkörper dichtend umgreift, **dadurch gekennzeichnet**, daß die Teilbereiche (4; 5, 6, 7) der Nadelschutzkappe (1, 2, 3) im Spritzgußverfahren in zumindest zwei Verfahrensschritten hergestellt werden, daß dazu in einem ersten Verfahrensschritt ein erster Teilbereich (4) der Nadelschutzkappe (1, 2, 3) hergestellt wird und daß in einem nachfolgenden Verfahrensschritt zumindest ein zweiter Teilbereich (5, 6, 7) hergestellt wird, wobei beim Einspritzen der Formmasse für den zweiten Teilbereich (5, 6, 7) in oder auf den ersten Teilbereich (4) der Nadelschutzkappe (1, 2, 3) dieser (4) mit dem zweiten Teilbereich (5, 6, 7) fest und untrennbar verschweißt oder dergleichen verbunden wird.

2. Verfahren nach Anspruch 1, dadurch gekennzeichnet, daß in einem ersten Verfahrensschritt der Stützkörper (4) hergestellt wird.

3. Verfahren nach Anspruch 1 oder 2, dadurch gekennzeichnet, daß die Nadelschutzkappe (1, 2, 3) im Zwei-Komponenten-Spritzgußverfahren hergestellt wird.

4. Nadelschutzkappe zum Aufstecken oder Aufschieben auf die Nadel und/oder den Spritzkörper einer Spritze, mit mindestens zwei Teilbereichen, von denen ein erster Teilbereich als steifer hülsenförmiger Stützkörper ausgebildet ist und von denen wengistens ein zweiter, aus elastischem Material bestehender Teilbereich im Hülseninneren des Stützkörpers angeordnet ist und in Gebrauchsstellung zumindest bereichsweise die Nadel und/oder den Spritzenkörper dicht umgreift, wobei diese beiden Teilbereiche der Nadelschutzkappe fest miteinander verbunden sind, dadurch gekennzeichnet, daß der als Stützkörper (4) vorgesehene Teilbereich aus einem thermoplastischen Kunststoff und der elastische Teilbereich (die elastischen Teilbereiche) (5, 6, 7) aus einem thermoplastischen Elastomer bestehen und daß diese Teilbereiche (4, 5, 6, 7) der Nadelschutzkappe (1, 2, 3) untrennbar miteinander verschweißt sind.

5. Nadelschutzkappe nach Anspruch 4, dadurch gekennzeichnet, daß der Stützkörper (4) in seinem Hülseninneren einen elastischen Teilbereich (5, 6, 7) trägt, der die Nadeln (12) zumindest im Bereich der Nadelspitze (9) und/oder den Spritzenkörper (11) in seinem an die Nadel angrenzenden Bereich (10) dicht umgreift.

6. Nadelschutzkappe nach Anspruch 4 oder 5, dadurch gekennzeichnet, daß die Nadelschutzkappe (1, 2, 3) einen Kappeninnenraum (13) mit einer Einstecköffnung (14) hat, welche (14) an einer Stirnseite der Nadelschutzkappe (1, 2, 3) angeordnet ist und daß der Kappeninnenraum (13) zumindest bereichsweise an die Nadeln (12) und/oder den Spritzenkörper (11) formangepaßt ist.

7. Nadelschutzkappe nach einem der Ansprüche 4 bis 6, dadurch gekennzeichnet, daß der Stützkörper (4) in Gebrauchsstellung der Nadelschutzkappe mit der Nadelspitze (9) abschließt oder diese überragt.

8. Nadelschutzkappe nach einem der Ansprüche 4 bis 7, dadurch gekennzeichnet, daß der hülsenförmige Stützkörper (4) an seiner der Einstecköffnung (14) abgewandten Stirnseite von einem elastischen Teilbereich (5, 7) dicht verschlossen ist, welcher vorzugsweise über das der Einstecköffnung (14) abgewandte Stirnende des Stützkörpers (4) zumindest bereichsweise übersteht.

9. Nadelschutzkappe nach einem der Ansprüche 4 bis 8, dadurch gekennzeichnet, daß die Einstecköffnung (14) durch einen elastischen Teilbereich (6, 7) begrenzt ist, der vorzugsweise über das der Einstecköffnung (14) zugewandte Stirnende des Stützelementes (4) übersteht.

10. Nadelschutzkappe nach einem der Ansprüche 4 bis 9, dadurch gekennzeichnet, daß der Kappeninnenraum (13) sich vorzugsweise in seinem zwischen einem Nadelansatzstück (10) des Spritzenkörpers (11) und der Nadelspitze (9) angeordneten Abschnitt (18) in Einsteckrichtung zumindest bereichsweise konisch verjüngt und daß der Kappeninnenraum (13) einen weiteren Abschnitt (19) mit im wesentlichen gleichbleibendem Querschnitt hat, welcher die Nadel (12) im Bereich der Nadelspitze (9) dicht umgreift.

11. Nadelschutzkappe nach einem der Ansprüche 4 bis 10, dadurch gekennzeichnet, daß im Kappeninnenraum (13) der Nadelschutzkappe (1, 2, 3) ein vorzugsweise umlaufender innerer Rastvorsprung (16) vorgsehen ist, der in Gebrauchsstellung in eine Einformung der Spritze (8) eingreift und/oder diese Einformung hintergreift.

12. Nadelschutzkappe nach einem der Ansprüche 4 bis 11, dadurch gekennzeichnet, daß der Rastvorsprung (16) als Ausformung eines elastischen Teilbereichs (6, 7) der Nadelschutzkappe (1, 2, 3) und die damit zusammenwirkende Einformung der Spritze (8) als eine vorzugsweise an die Nadel (12) angrenzende und insbesondere wulstförmige Querschnittserweiterung (15) des Nadelansatzstückes (10) ausgebildet ist.

13. Nadelschutzkappe nach einem der Ansprüche 4 bis 12, dadurch gekennzeichnet, daß der Kappeninnenraum (13) in seinem die Einstecköffnung (14) begrenzenden Bereich einen im Vergleich zum Außendurchmesser des in Gebrauchsstellung benachbarten Spritzenkörper-Bereichs geringfügig größeren lichten Durchmesser hat.

14. Nadelschutzkappe nach einem der Ansprüche 4 bis 13, dadurch gekennzeichnet, daß der konisch verjüngte Abschnitt (18) des Kappeninnenraums (13) durch eine vorzugsweise flanschartige Einformung (20) im Hülseninneren des Stützkörpers gebildet ist und daß dieses Einformung (20) des Stützkörpers (4) in seinem Hülseninneren zwei elastische Teilbereiche (5, 6) voneinander trennt.

15. Nadelschutzkappe nach einem der Ansprüche 4 bis 14, dadurch gekennzeichnet, daß der Stützkörper (4) in seinem Hülseninneren mit zwei elastischen Teilbereichen (5, 6) untrennbar verschweißt ist, von denen ein erster Teilbereich (6) den Spritzenkörper (11) vorzugsweise im Bereich des Nadelansatzstückes (10) und von denen ein zweiter Teilbereich (5) die Nadel (12) zumindest bereichsweise dicht umgreift und daß diese beiden elastischen Teilbereiche (5, 6) vorzugsweise über einen aus demselben thermoplastischen Elastomer bestehenden Außenmantel (21) miteinander verbunden sind, welcher den Stützkörper (4) außenseitig umgibt.

16. Nadelschutzkappe nach einem der Ansprüche 4 bis 15, dadurch gekennzeichnet, daß die Nadelschutzkappe (3) einen einstückigen elastischen Teilbereich (7) aufweist und daß das Stützelement (4) diesen Teilbereich (7) zumindest bereichsweise hülsenförmig umgreift und vorzugsweise im wesentlichen den Außenmantel der Nadelschutzkappe (3) bildet.
